Europäisches Patentamt

European Patent Office

Office européen des brevets

(19)

(11) Veröffentlichungsnummer: **0 281 855**
**B1**

(12) **EUROPÄISCHE PATENTSCHRIFT**

(45) Veröffentlichungstag der Patentschrift: **06.02.91**

(51) Int. Cl.⁵: **A 61 F 2/54**

(21) Anmeldenummer: **88102776.7**

(22) Anmeldetag: **25.02.88**

(54) **Innenhand.**

(30) Priorität: **03.03.87 DE 3706800**

(43) Veröffentlichungstag der Anmeldung:
**14.09.88 Patentblatt 88/37**

(45) Bekanntmachung des Hinweises auf die
Patenterteilung:
**06.02.91 Patentblatt 91/06**

(84) Benannte Vertragsstaaten:
**AT BE CH DE ES FR GB GR IT LI LU NL SE**

(56) Entgegenhaltungen:
**DE-A-2 801 299**
**DE-B-1 049 534**
**FR-A-2 414 326**
**US-A-4 521 924**

(73) Patentinhaber: **Otto Bock Orthopädische
Industrie Besitz- und Verwaltungs-
Kommanditgesellschaft
Industriestrasse
D-3408 Duderstadt 1 (DE)**

(72) Erfinder: **Gammer, Peter
Auhofstrasse 249/3
A-1130 Wien (AT)**

(74) Vertreter: **Gramm, Werner, Prof. Dipl.-Ing. et al
Patentanwälte Gramm + Lins Theodor-Heuss-
Strasse 2
D-3300 Braunschweig (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Innenhand als orthopädisches Paßteil aus thermoplastischem Kunststoff zur Aufnahme einer Handmechanik, mit am Handabschluß eingearbeiteter Ringnut, in die ein die Innenhand auf der Handmechanik fixierender Ring eingelegt ist. Eine derartige Innenhand ist aus der DE—A—2 801 299 bekannt. Diese vorbekannte Innenhand (DE—A—2 801 299) ist aus PVC gefertigt, weist eine gleichmäßig durchgehende Wandstärke mit gleicher Materialhärte auf und dient zur formgebenden Verkleidung der Handmechanik. Die Ringnut ist als Rundnut ausgeführt, in die ein Wulstring eingelegt wird.

Form- und Proportionsgestaltung sind verantwortlich für Materialspannungen, die insbesondere im Zwischenbereich von Daumen und Zeigefinger sowie an der Handrückenpartie auftreten. Hier liegt die Ursache für einen unökonomischen Energieverbrauch in Verbindung mit einer reduzierten Öffnungsweite zwischen Daumen und Zeigefinger. An den Nutzungsstellen von Daumen, Zeige- und Mittelfinger sowie im Zwischenbereich von Daumen und Zeigefinger erhält man nur eine geringe mechanische Festigkeit. Jedoch führt die materialspezifische Formstabilität insbesondere im Handabschluß zu hubartigen Volumenverschiebungen, so daß kein wirksamer Schutz der Mechanik gegen Schweiß und sonstige Verunreinigung gewährleistet ist. Bedingt durch das homogene, in sich feste und unflexible PVC-Material ergibt sich ein relativ schlechter Wirkungsgrad mit den vorstehend genannten Nachteilen, die bischer jedoch als vertretbarer Kompromiß in Kauf genommen wurden.

Der Erfindung liegt die Aufgabe zugrunde, die Funktion der eingangs beschriebenen Innenhand zu verbessern.

Diese Aufgabe wird erfindungsgemäß dadurch gelöst, daß der Nutzungsbereich zumindest des Daumens und Zeigefingers einen höheren Härtegrad aufweist aus der übrige Handkörper, und daß im Handkörperbereich zwischen Daumen und Zeigefinger Rippen vorgesehen sind, zwischen denen die Wandstärke des Handkörpers geringer ist als im übrigen Handkörperbereich.

Dabei ist es zweckmäßig, wenn der genannte Nutzungsbereich aus kompaktem Kunststoff, der übrige Handkörper aber aus geschlossenzelligem Schaumstoff besteht.

Es handelt sich also um eine Zweikomponenten-Sandwich-Bauweise, die auch als Hybrid-Bauweise bezeichnet werden kann. Die erfindungsgemäße Innenhand weist also eine scharfe Abgrenzung zwischen den beiden genannten Bereichen auf, wobei die Shorehärte des genannten Nutzungsberiches etwa 80, die des übrigen Handkörpers etwa 50 beträgt. Die erfindungsgemäße Innenhand besteht also aus zwei Materialien mit unterschiedlichen Härtegraden und physikalischen Eigenschaften.

Eine neue Form- und Proportionsgestaltung führt in Verbindung mit dem flexibel aufgeschäumten PVC zu einer spannungsfreien, energiesparenden und vollständigen Öffnungsweite zwischen Daumen und Zeigefinger. Das härter eingestellte Material in dem genannten Nutzungsbereich erhöht die mechanische Verschleißfestigkeit und dient als Maßnahme zur Verhinderung von Kaltfluß in diesen Zonen.

Durch die Anordnung der Rippen wird der Öffnungsbereich zwischen Daumen und Zeigefinger noch weiter verbessert; gleichzeitig wird aber eine hohe Verschleißfestigkeit sichergestellt.

Die Oberfläche der genannten Rippen bildet eine verschleißfeste Zone zwischen Daumen und Zeigefinger, wobei die reduzierte Wandstärke zwischen den Rippen eine ausreichende Flexibilität garantiert. Die Anordnung der Rippen führt überdies dazu, daß sich die Rippen radiusförmig aneinander anlegen können, so daß sich beim Zusammenführen von Daumen und Zeigefinger eine kraftarme Materialverformung ergibt.

Um die Mechanik wirkungsvoller gegen Schweiß und sonstige Verunreinigungen zu schützen, können im Handabschluß zwei Ringnuten vorgesehen sein, in die Ringstege eines den Handabschluß im Bereich beider Ringnuten umgreifenden Ringes eingreifen. Dabei ist es vorteilhaft, wenn die außenliegende Ringnut tiefer ausgebildet ist als die innenliegende Ringnut, und daß der der außenliegenden Ringnut zugeordnete Ringsteg entsprechend breiter ist als der andere Ringsteg.

Die vom Ringinnendurchmesser sowie durch die Anordnung der Ringstege vorbestimmte axialen und radialen Kräfte ergeben einen optimalen Anpreßdruck auf das abzudichtende Chassi der Handmechanik. Zugleich ergibt sich eine Verbesserung der mechanischen Sicherung der Innenhand auf der Handmechanik.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:

Figur 1 in schaubildlicher Darstellung eine Innenhand;

Figur 2 einen Längschnitt in der durch Daumen und Zeigefinger gebildeten Ebene und

Figur 3 einen Querschnitt durch einen Ring.

Bei der dargestellten Innenhand handelt es sich um ein aus thermoplastichem Kunststoff hergestelltes orthopädisches Paßteil zur Aufnahme einer Handmechanik. Finger- und Knöckelbereiche sind kosmetisch ausdrucksvoll gestaltet. Bei Benutzung dieser Innenhand durch den Prosthesenträger wird die Innenhand üblicherweise von einem Kosmetik-Handschuh abgedeckt. Die Innenhand wird am Handabschluß 1 an einer nicht näher dargestellten Handmechanik befestigt, die in die Innenhand ragt und die Bewegung zumindest des Daumens und des Zeigefingers steuert.

Erfindungsgemäß besteht die Innenhand aus einer Kombination zweier Materialien mit unterschiedlichen Härtegraden und physikalischen Eigenschaften. Dabei besteht der Nutzungsbereich 2 deu Daumens 3 sowie des Zeigefingers 4 und des Mittelfingers 5 aus einem festen kompakten thermoplastichem Kunststoff, vorzugsweise PVC, mit einer Shorehärte von etwa 80. Der sich an diesen Nutzungsbereich 2 unmittelbar anschließende übrige Handkörper 6 besteht aus einem weicheren Werkstoff mit Blasenstruktur und ist als geschlossenzelliger Schaumstoff mit einer Shorehärte von etwa 50 ausgebildet. Es handelt sich also um eine sogenannte Zweikomponenten-Sandwich-Bauweise bzw. um eine Hybrid-Bauweise.

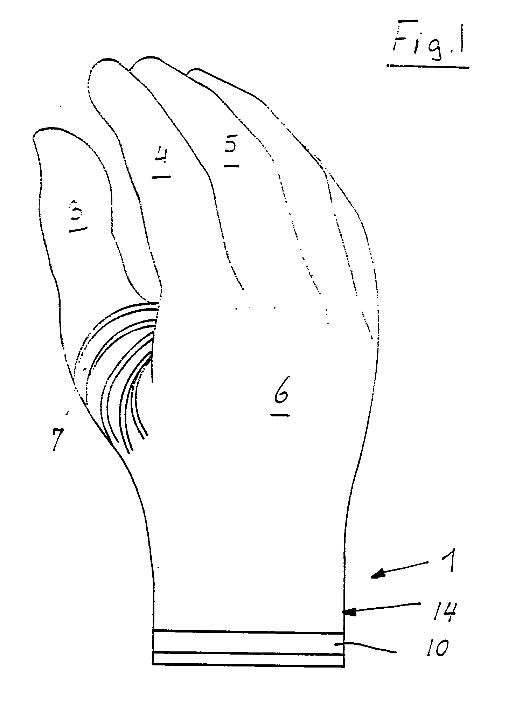

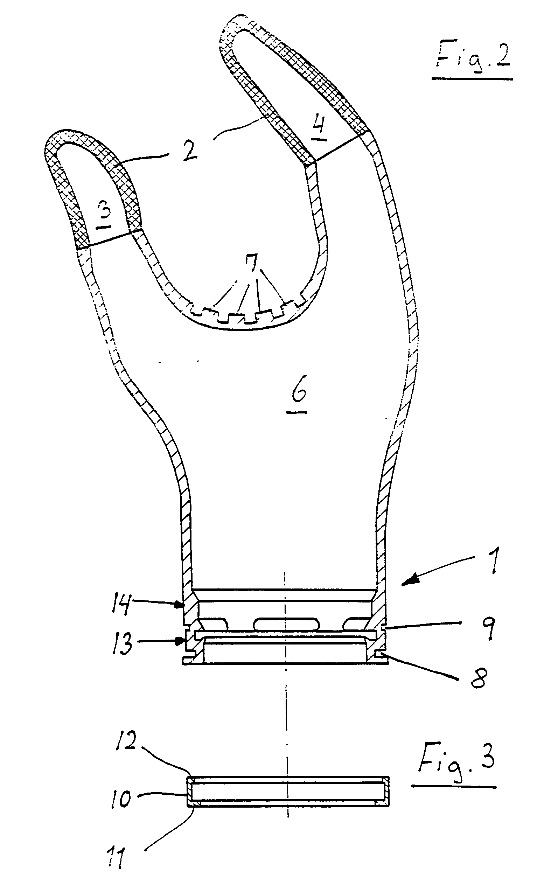

Im Handkörperbereich zwischen Daumen 3 und Zeigefinger 4 sind Rippen 7 vorgesehen, die angenähert parallel zueinander verlaufen und jeweils in Ebenen liegen, die senkrecht auf der durch Daumen 3 und Zeigefinger 4 definierten Ebene stehen, die die Zeichnungsebene der Figur 2 ist. Figur 2 läßt erkennen, daß die Wandstärke des Handkörpers 6 zwischen den genannten Rippen 7 geringer ist als im übrigen Handkörperbereich.

Der hart eingestellte Nutzungsbereich 2 sowie die Rippen 7 zwischen Daumen und Zeigefinger sind stabilisierende Maßnahmen gegen mechanischen Verschleiß. Anordnung und Ausbildung der Rippen 7 sind so gewählt, daß sich beim Zusammenführen von Daumen 3 und Zeigefinger 4 die Rippen radiusförmig aneinander legen können, wodurch eine kraftarme Materialverformung sichergestellt wird.

Im Handabschluß 1 sind zwei Ringnuten 8, 9 vorgesehen, von denen die außenliegende Ringnut 8 tiefer ausgebildet ist als die innenliegende Ringnut 9. Beide Ringnuten 8, 9 sind von einem Ring 10 übergriffen, der mit Ringstegen 11, 12 in die Ringnuten 8, 9 eingreift, wobei der der außenliegenden Ringnut 8 zugeordnete Ringsteg 11 entsprechend breiter ist als der andere Ringsteg 12. In die Oberfläche des Handabschlusses 1 ist eine den Ring 10 aufnehmende ringförmige Aussparung 13 eingearbeitet, so daß der Ring 10 nahezu bündig in der Oberfläche 14 des Handabschlusses 1 liegt. Dies läßt Figur 1 erkennen, während in Figur 2 aus Gründen der bessern Übersichtlichkeit der Ring 10 weggelassen wurde. Die Ausbildung des Ringes zeigt Figur 3.

## Patentansprüche

1. Innenhand als orthopädisches Paßteil aus thermoplastischem Kunststoff zur Aufnahme einer Handmechanik, mit am Handabschluß (1) eingearbeiteter Ringnut (8, 9), in die ein die Innenhand auf der Handmechanik fixierender Ring (10) eingelegt ist, dadurch gekennzeichnet, daß der Nutzungsbereich (2) zumindest des Daumens (3) und Zeigefingers (4) einen höheren Härtegrad aufweist als der übrige Handkörper (6), und daß im Handkörperbereich zwischen Daumen (3) und Zeigefinger (4) Rippen (7) vorgesehen sind, zwischen denen die Wandstärke des Handkörpers (6) geringer ist als im übrigen Handkörperbereich.

2. Innenhand nach Anspruch 1, dadurch gekennzeichnet, daß der genannte Nutzungsbereich (2) aus kompaktem Kunststoff, der übrige Handkörper (6) aber aus geschlossenzelligem Schaumstoff besteht.

3. Innenhand nach Anspruch 1 oder 2, dadurch gekennzeichnet, daß die Shorehärte des genannten Nutzungsbereichs (2) etwa 80, die des übrigen Handkörpers (6) etwa 50 beträgt.

4. Innenhand nach Anspruch 1, 2 oder 3, dadurch gekennzeichnet, daß die genannten Rippen (7) angenähert parallel zueinander verlaufen und jeweils in Ebenen liegen, die senkrecht auf der durch Daumen (3) und Zeigefinger (4) definierten Ebene stehen.

5. Innenhand nach einem der vorhergehenden Ansprüche, dadurch gekennzeichnet, daß im Handabschluß (1) zwei Ringnuten (8, 9) vorgesehen sind, in die Ringstege (11, 12) eines den Handabschluß (1) im Bereich beider Ringnuten (8, 9) umgreifenden Ringes (10) eingreifen.

6. Innenhand nach Anspruch 5, dadurch gekennzeichnet, daß die außenliegende Ringnut (8) tiefer ausgebildet ist als die innenliegende Ringnut (9), und daß der der außenliegenden Ringnut (8) zugeordnete Ringsteg (11) entsprechend breiter ist als der andere Ringsteg (12).

7. Innenhand nach Anspruch 5 oder 6, dadurch gekennzeichnet, daß in die Oberfläche des Handabschlusses (1) eine den Ring (10) aufnehmende ringförmige Aussparung (13) eingearbeitet ist, so daß der Ring (10) nahezu bündig in der Oberfläche (14) des Handabschlusses (1) liegt.

## Revendications

1. Main interne sous forme d'une pièce d'adaptation orthopédique fabriquée en matière thermoplastique et destinée à recevoir un mécanisme de main, qui comporte une gorge annulaire (8, 9) ménagée à l'extrémité (1) de la main, dans laquelle est inséré le bracelet (10) fixant la main interne au mécanisme de main, caractérisée en ce que la zone d'utilisation (2) d'au moins le pouce (3) et l'index (4) présente une plus grande dureté que le reste du corps (6) de la main et que, dans la zone du corps de la main qui est comprise entre le pouce (3) et l'index (4), sont prévues des nervures (7) entre lesquelles l'épaisseur de paroi du corps (6) de la main est inférieure à celle du reste du corps de la main.

2. Main interne suivant la revendication 1, caractérisée en ce que la zone d'utilisation (2) est faite d'une matière plastique compacte et le reste du corps (6) de la main est constitué d'une matière expansée à cellules fermées.

3. Main interne suivant la revendication 1 ou 2, caractérisée en ce que la dureté Shore de la zone d'utilisation (2) est d'environ 80 et celle du reste du corps (6) de la main d'environ 50.

4. Main interne suivant la revendication 1, 2 ou 3, caractérisée en ce que les nervures (7) sont à près parallèles l'une à l'autre et sont chaque fois situées dans un plan qui est perpendiculaire au plan défini par le pouce (3) et l'index (4).

5. Main interne suivant l'une quelconque des revendications précédentes, caractérisée en ce que l'extrémité (1) de la main présente deux gorges annulaires (8, 9), dans lesquelles s'engagent les languettes annulaires (11, 12) d'un bracelet (10) entourant l'extrémité (1) de la main dans la zone des deux gorges annulaires (8, 9).

6. Main interne suivant la revendication 5, caractérisée en ce que la gorge annulaire externe (8) est plus profonde que la gorge annulaire interne (9) et que la languette annulaire (11) associée à la gorge annulaire externe (8) est de manière correspondante plus large que l'autre languette annulaire (12).

7. Main interne suivant la revendication 5 ou 6, caractérisée en ce qu'un creux annulaire (13) recevant le bracelet (10) est ménagé dans la surface de l'extrémité (1) de la main, de sorte que le bracelet (10) affleure approximativement la surface (14) de l'extrémité (1) de la main.

**Claims**

1. Internal hand as an orthopaedic fitting part of thermoplastic synthetic material for receiving a hand mechanism, having an annular groove (8, 9) which is made in the hand end (1) and in which there is inserted a ring (10) fixing the internal hand on the hand mechanism, characterised in that the region of use (2) at least of the thumb (3) and the index finger (4) has a greater degree of hardness than the remaining hand body (6), and in that there are provided in the hand body region between the thumb (3) and the index finger (4) ribs (7) between which the wall thickness of the hand body (6) is less than in the remaining region of the hand body.

2. Internal hand according to Claim 1, characterised in that the said region of use (2) comprises compact synthetic material but the remaining hand body (6) comprises closed-cell foam material.

3. Internal hand according to Claim 1 or 2, characterised in that the Shore hardness of the said region of use (2) is approximately 80 and that of the remaining hand body (6) is approximately 50.

4. Internal hand according to Claim 1, 2 or 3, characterised in that the said ribs (7) run approximately parallel to one another and are respectively in planes perpendicular to the plane defined by the thumb (3) and the index finger (4).

5. Internal hand according to one of the precedicg claims, characterised in that there are provided in the hand end (1) two annular grooves (8, 9), in which annular webs (11, 12) of a ring (10) surrounding the hand end (1) in the region of both annular grooves (8, 9) engage.

6. Internal hand according to Claim 5, characterised in that the outer annular groove (8) is made deeper than the inner annular groove (9), and in that the annular web (11) associated with the outer annular groove (8) is correspondingly larger than the other annular web (12).

7. Internal hand according to Claim 5 or 6, characterised in that an annular recess (13) receiving the ring (10) is made in the surface of the hand end (1) so that the ring (10) lies almost flush with the surface (14) of the hand end (1).

Fig.1

3

4

5

7

6

1

14

10

1

_Fig.2_

2

_4_

_3_

7

_6_

1

14

13

9

8

12

10

11

_Fig.3_